# EUROPÄISCHE PATENTSCHRIFT

(11) **EP 2 386 287 B1**
(45) Veröffentlichungstag und Bekanntmachung des Hinweises auf die Patenterteilung: **12.07.2017**
(21) Anmeldenummer: 11165581.7
(22) Anmeldetag: 10.05.2011
(51) Int. Cl.: A61K 6/10

(54) **Tenside enthaltende Zusammensetzung zur dentalen Abformung**
Compound for dental casting containing tensides
Composé comprenant des agents tensioactifs pour la prise d'empreinte dentaire

(30) Priorität: 12.05.2010 DE 102010028973
(43) Veröffentlichungstag der Anmeldung: 16.11.2011
(73) Patentinhaber: VOCO GmbH, 27472 Cuxhaven (DE)
(72) Erfinder: Riedel, Norman Hendrik, 60435 Frankfurt am Main (DE); Plaumann, Manfred Thomas, 27476 Cuxhaven (DE)
(74) Vertreter: Eisenführ Speiser

(56) Entgegenhaltungen:
- EP-A2- 2 253 302
- WO-A2-2009/079534
- US-A1- 2008 319 100
- US-B1- 6 291 546

## Beschreibung

Die vorliegende Erfindung betrifft eine in den Ansprüchen weiter spezifizierte aushärtbare, ein- oder mehrkomponentige Zusammensetzung zur dentalen Abformung (nachfolgend auch dentale Abformmaterialien genannt), umfassend
- eine aushärtbare Basiszusammensetzung
   und
- ein Tensidsystem umfassend oder bestehend aus
   - 0,5 bis 3 Gew.-%, eines ersten Tensids, welches ausgewählt ist aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen,
   - 0,5 bis 3 Gew.-% eines zweiten Tensids, welches ausgewählt ist aus der Gruppe der nichtionischen Fluortenside,
   - 0 bis 3 Gew.-% eines dritten Tensids, welches ausgewählt ist aus der Gruppe der Silikontenside,
   - wobei die Gesamtmenge an erstem, zweitem und drittem Tensid im Bereich von 1,5 Gew.-% bis 6 Gew.-% liegt,
   mit der Maßgabe, dass
   - das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% oder weniger beträgt,
   wobei sämtliche Gewichtsprozentangaben bezogen sind auf die Gesamtmasse der Zusammensetzung.

Die Erfindung betrifft auch die Verwendung einer erfindungsgemäßen Zusammensetzung als dentales Abformsystem sowie ein Verfahren zur Herstellung einer erfindungsgemäßen Zusammensetzung.

Das Benetzungsverhalten eines dentalen Abformmaterials gegenüber Speichel ist einer der kritischen Parameter für die Präzision der erhaltenen dentalen Abformung. Herkömmliche Abformmaterialien, zum Beispiel Abformmaterialien auf Polydimethylsiloxan-Basis weisen allerdings ein ungenügendes Benetzungsverhalten gegenüber wässrigen Medien auf, so dass in manchen Fällen Kontaktwinkel von 90 ° und mehr resultieren. Zur Optimierung des Anfließverhaltens dentaler Abformmaterialien in hydrophiler Umgebung werden daher seit vielen Jahren Tensidzusätze vorgeschlagen und verwendet.

US 4,657,959 offenbart aushärtbare Silikonzusammensetzungen, die ein Tensid, insbesondere ein Fluortensid, enthalten, als dentale Abformmaterialien. Wird auf der Oberfläche eines solchen Abformmaterials ein Wassertropfen abgesetzt, so stellt sich gemäß der US-Schrift nach drei Minuten ein Kontaktwinkel von weniger als 10 ° ein.

EP 1 290 998 B1 offenbart eine Zusammensetzung zur dentalen Abformung auf Basis additionsvernetzender Organopolysiloxane, wobei die Zusammensetzung ein nichtionisches Tensid und/oder ein Polyether-modifiziertes Silikonöl enthält.

In WO 2004/058 196 A1 wird ein dentales Abdruckmaterial auf Basis von Polyvinylsiloxan beschrieben, wobei durch ein nichtionisches Tensid, insbesondere PEG8-Methicone, 15 Minuten nach Aushärten innerhalb von 15 Sekunden ein Kontaktwinkel kleiner 10 ° erreicht werden soll. Ein solches Benetzungsverhalten ist allerdings nur für das Anfließverhalten beim Gipsabdruck relevant, nicht jedoch für die Abdrucknahme im Mund eines Patienten.

EP 0 729 341 B1 betrifft ein dentales Abformmaterial, das zur Hydrophilierung Polyethercarbosilane enthält. Hierdurch werden lediglich Kontaktwinkel von 42° oder mehr erreicht.

In EP 0 231 420 B1 wird die Hydrophilierung eines dentalen Abformmaterials auf Basis additionsvernetzender Silicone durch Silikonpolyether beschrieben.

Auch in US 5,064,891 A wird eine ein Tensid enthaltende dentale Abformmasse offenbart. Vergleiche auch EP 2 253 302 A2.

In US 5,907,002 A werden dentale Abdruckmassen beschrieben, die ein nichtionisches Tensid enthalten. Als Beispiele für die nichtionischen Tenside werden Polyoxyethylenalkylether, Polyoxypropylenalkylether, Polyoxyethylenalkylphenylether sowie verschiedene Fettsäureester und Fluoralkylethylenoxidtenside angegeben.

In DE 199 22 929 A1 und der entsprechenden US 6,291,546 B1 werden Zusammensetzungen zur dentalen Abdrucknahme der Mundschleimhaut beschrieben, die ein oder mehrere nichtionische Tenside enthalten. Als Beispiele für die nichtionischen Tenside werden Polyoxyethylenalkylether, Poly-oxypropylenalkylether, Polyoxyethylenalkylphenylether, insbesondere Polyoxyethylennonylphenylether; sowie verschiedene Fettsäureester und Fluoralkyl-ethylenoxidtenside angegeben. Massen, die sowohl einen dieser Ether als auch ein Fluortensid enthalten, werden nicht offenbart.

In DE 10 2006 001 126 A1 werden Zusammensetzungen für dentale Abformmaterialien beschrieben, die neben einem härtbaren Polymer mindestens ein nichtionisches Tensid mit einer Molmasse von weniger als 6000 g/mol, das einen (Poly)alkylenoxidrest sowie eine Silizium enthaltende Gruppe aufweist (im folgenden als Silizium aufweisendes Tensid bezeichnet), und mindestens ein nichtionisches Fluortensid, das einen (Poly)-alkylenoxidrest aufweist und mindestens einen teil- oder perfluorierten Kohlenwasserstoffrest, der über ein Sauerstoffatom oder eine Estergruppe mit dem (Poly)alkylenoxidrest verbunden ist (im folgenden als Fluortensid bezeichnet) enthalten. Das Gewichtsverhältnis von Silizium aufweisendem Tensid zu Fluortensid liegt im Bereich von 100:1 bis 1:100, bevorzugt 50:1 bis 1:50, besonders bevorzugt 10:1 bis 1:10 und insbesondere bevorzugt 5:1 bis 1:5. In bevorzugten Zusammensetzungen beträgt der Gehalt
der Gehalt an Silizium aufweisendem Tensid und Fluortensid, bezogen auf die Gesamtmasse der Zusammensetzung, jeweils 0,001 bis 10 Gew.-%. Zusätzlich zu dem vorstehend beschriebenem Silizium enthaltenden Tensid und dem vorstehend beschriebenem Fluortensid kann die Zusammensetzung weitere Tenside enthalten, u.a. alkyl-, aryl-, aralkyl-verschlossene nichtionische Tenside, bevorzugt alkyl-verschlossene Fettalkoholethoxylate, Silicontenside, Polyethercarbosilane, Carbosilantenside und Fluortenside, die alkyl-verschlossen sind und insbesondere alkylverschlossene Fettalkoholethoxylate.

Die aus den in der Patentanmeldung DE 10 2006 001 126 A1 offenbarten Zusammensetzungen erhältlichen dentalen Abformmaterialien sollen einen initialen Kontaktwinkel, (gemessen zwischen 3 und 10 Sekunden Tropfenalter, wobei die Aufgabe des Wassertropfens 40 Sekunden nach Beginn des Mischens der Katalysator- und der Basiskomponente des härtbaren Polymersystems erfolgt) von < 10° aufweisen, und während der Verarbeitungszeit zwischen 0 und 3 Minuten einen Gleichgewichtskontaktwinkel von < 10°. In Vergleichsbeispielen wurden auch Zusammensetzungen getestet, die ein Fluortensid (0,75 bzw. 1,5 Gew-%, bezogen auf die Gesamtmasse der Zusammensetzung) und ein nichtionisches alkylverschlossenes Fettalkoholethoxylat-Tensid mit einer Oberflächenspannung von 29 mN/m (0,2 Gew-%, bezogen auf die Gesamtmasse der Zusammensetzung), jedoch kein Silizium aufweisendes Tensid enthalten. Die so erhaltenen Abformmaterialien werden jedoch als nicht ausreichend hydrophil beschrieben, d.h. innerhalb von 30 Sekunden nach Aufgabe eines Wassertropfens stellte sich ein Kontaktwinkel ein, der deutlich größer als 10° ist. Zusammensetzungen enthaltend 0,2 Gew.-% eines nichtionisches alkylverschlossenen Fettalkoholethoxylat-Tensids sollen nur dann Abformmaterialien mit akzeptablen Benetzungsverhalten geliefert haben, wenn die Zusammensetzung außerdem 0,75 Gew.-% Fluortensid und 0,75 Gew.-% Silizium aufweisendem Tensid bzw. 0,375 Gew.-% Fluortensid und 1,125 Gew.-% Silizium aufweisendem Tensid enthielt.

DE 10 2009 021 553 A1 offenbart eine härtbare Zusammensetzung enthaltend härtbare Polymere und ferner mindestens ein nichtionisches und/oder ionisches Fluortensid ausgewählt aus einer Gruppe bestehend aus acht speziellen Klassen von Fluortensiden. Die Zusammensetzung kann zusätzlich mindestens ein weiteres Fluortensid und/oder mindestens ein Silicium aufweisendes nichtionisches Tensid mit einer Molmasse von weniger als 6000 g/mol und/oder einen Alkenylreste und/oder Alkinylreste enthaltenden Polyether und/oder einen hydroxyl- und/oder aryloxy- und/oder arylalkyloxy- und/oder DE 43 06 997 A1 offenbart eine gummielastische Masse, z.B. eine dentale Abformmasse, auf der Basis vulkanisierbarer Polyethermaterialien, die wenigstens ein hydrophiles Silikonöl und/oder ein nicht-ionisches grenzflächenaktives Mittel enthält. Für das nichtionische grenzflächenaktive Mittel werden zahlreiche Alternativen aus den verschiedensten Tensidklassen vorgeschlagen, u.a. fluorierte Kohlenwasserstoffverbindungen sowie alkoxylierte und acylierte alkoxylierte Fettalkohole. Massen, die sowohl einen alkoxylierten Fettalkohol als auch ein Fluortensid enthalten, werden nicht offenbart. Alle beispielhaft beschriebenen Abformmassen enthalten jeweils nur ein einzelnes Tensid. Die gummielastischen Massen sollen im fertig vulkanisierten und auspolymerisierten Zustand einen 10-Sekunden-Randwinkel von weniger als 55°, vorzugsweise weniger als 45° und insbesondere weniger als 35° aufweisen. Der Randwinkel der auspolymerisierten Masse lässt keinen Rückschluss darauf zu, wie gut diese Abformmasse bei der Zahnabdrucknahme, also im noch nicht ausgehärteten Zustand, die feuchte, mit Speichel und gegebenenfalls Blut bedeckte Zahnsubstanz und Mundschleimhaut benetzt und auf diese auffließt.

WO 2009/079534 A2 offenbart eine härtbare dentale Zusammensetzung umfassend ein oder mehrere Tenside und eine fluorhaltige Verbindung der dort angegebenen Formel. Das Tensid kann z.B. ein Silizium enthaltendes Tensid sein oder ein ethoxylierter Fettalkohol. Dabei wurde festgestellt, dass dentale Zusammensetzungen, die eine Fluorverbindung wie in WO 2009/079534 A2 definiert und kein Tensid enthalten, kein verbessertes Benetzungsverhalten aufweisen im Vergleich zu Zusammensetzungen, die ein typisches Fluortensid wie z.B. Zonyl^{™} FSO 100 enthalten, während andererseits dentale Zusammensetzungen enthaltend eine Fluorverbindung wie in WO 2009/079534 A2 definiert in Kombination mit einem Tensid ein verbessertes Benetzungsverhalten aufweisen.

Auf dem Gebiet der Dentaltechnik besteht ein ständiger Bedarf an Zusammensetzungen für dentale Abformmaterialien, die hinreichend hydrophil sind, um bei der Zahnabdrucknahme, also im noch nicht ausgehärteten Zustand des Abformmaterials, sofort, vorzugsweise spontan die feuchte, mit Speichel und ggf. Blut bedeckte Zahnsubstanz und Mundschleimhaut zu benetzen und unmittelbar auf diese auffließen, so dass ein detailgetreuer Abdruck der dentalen Situation erhalten wird.

Es war die Aufgabe der vorliegenden Erfindung, eine derartige geeignete Zusammensetzung zur dentalen Abformung bereitzustellen. Erfindungsgemäße Zusammensetzungen sind in den beigefügten Ansprüchen definiert.

Beschrieben wird in diesem Zusammenhang auch eine aushärtbare, ein- oder mehrkomponentige Zusammensetzung zur dentalen Abformung, umfassend
- eine aushärtbare Basiszusammensetzung
und
- ein Tensidsystem umfassend oder bestehend aus
   - 0,5 bis 3 Gew.-%, eines ersten Tensids, welches ausgewählt ist aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen,
   - 0,5 bis 3 Gew.-% eines zweiten Tensids, welches ausgewählt ist aus der Gruppe der nichtionischen Fluortenside,
   - 0 bis 3 Gew.-% (d.h. 0 Gew.-% oder eine Menge bis zu 3 Gew.-%) eines dritten Tensids, welches ausgewählt ist aus der Gruppe der Silikontenside,
   - wobei die Gesamtmenge an erstem, zweitem und drittem Tensid im Bereich von 1,5 Gew.-% bis 6 Gew.-% liegt,
   mit der Maßgabe, dass
   - das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% oder weniger beträgt,
wobei sämtliche Gewichtsprozentangaben bezogen sind auf die Gesamtmasse der Zusammensetzung.

Beschrieben wird zudem eine aushärtbare Zusammensetzung der oben beschriebenen Art umfassend
- eine aushärtbare Basiszusammensetzung
und
- ein Tensidsystem umfassend
   - ein erstes Tensid ausgewählt aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen,
   - ein zweites Tensid ausgewählt aus der Gruppe der nichtionischen Fluortenside,
   - optional ein drittes Tensid ausgewählt aus der Gruppe der Silikontenside,
   mit der Maßgabe, dass das Tensidsystem
   - 0,75 bis 3 Gew.-%, des zweiten Tensids enthält,
   - 0 bis weniger als 0,5 Gew.-% des dritten Tensids enthält,
   oder das Tensidsystem
   - 0,5 bis 2,5 Gew.-% des ersten Tensids enthält,
   - 0,5 bis 2,5 Gew.-% vorzugsweise 0,75 bis 2,5 Gew.-% des zweiten Tensids enthält
   - 0,5 bis 2,5 Gew.-% des dritten Tensids enthält.

Überraschend hat sich in eigenen Untersuchungen gezeigt, dass eine vorstehend beschriebene Zusammensetzung beim Aushärten das gewünschte Benetzungsverhalten aufweist. Eine besonders bevorzugte beschriebene Zusammensetzung weist beim Aushärten ein derartiges Benetzungsverhalten auf, dass sich bei einem 40 Sekunden nach Beginn des Aushärtens auf die Oberfläche des aushärtenden Abformmaterials applizierten Wassertropfen in kurzer Zeit, d.h. innerhalb von 30 Sekunden oder weniger, vorzugsweise innerhalb von 10 Sekunden oder weniger, ein Kontaktwinkel von 10° einstellt.

Bei einem Gesamtgehalt an erstem, zweitem und drittem Tensid von weniger als 1,5 Gew.-% ist die Hydrophilierung nicht ausreichend, d.h. bei Aufgabe eines Wassertropfens auf das aushärtende Abformmaterial stellt sich nie oder zumindest nicht nach kurzer Zeit ein Kontaktwinkel ein, der kleiner als 10 ° ist.

Ein Gesamtgehalt an erstem, zweitem und drittem Tensid von mehr als 6 Gew.-% beeinflusst das Abbindeverhalten des aushärtbaren Basissystems ungünstig, das System härtet dann sehr langsam und eventuell unvollständig aus. Grund dafür ist u.a. die zunehmende Komplexierung des Katalysators durch die Tenside.

Das in der beschriebenen Zusammensetzung einzusetzende erste Tensid ist ausgewählt aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen. "Endgruppenverschlossen" bedeutet hierbei, dass in den endständigen Hydroxylgruppen die Wasserstoffatome durch Alkylgruppen substituiert sind. Vorzugsweise sind in den endständigen Hydroxylgruppen die Wasserstoffatome durch Alkylgruppen umfassend ein bis sechs Kohlenstoffatome, besonders bevorzugt mit Alkylgruppen umfassend ein bis drei Kohlenstoffatome substituiert. Bevorzugt sind auch endgruppenverschlossene Polyoxyethylenalkylether, endgruppenverschlossene Polyoxypropylenalkylether, endgruppenverschlossene Polyoxyethylenpolyoxypropylenalkylether, bei denen eine erste endständige Hydroxylgruppe durch eine Alkylgruppe mit 6 bis 23 Kohlenstoffatomen und eine zweite endständige Hydroxylgruppe durch eine Alkylgruppe mit 1 bis 6, vorzugsweise 1 bis 3 Kohlenstoffatomen ersetzt ist, und deren Mischungen.

Erfindungsgemäß ist das erste Tensid eine Verbindung der Formel 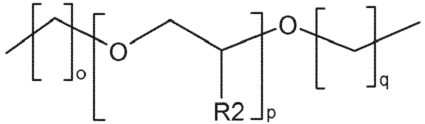 worin
o eine ganze Zahl von 5 bis 22 ist,
p eine ganze Zahl von 2 bis 20 ist, und
q eine ganze Zahl von 0 bis 5 ist,
R2 Wasserstoff oder eine Methylgruppe ist, wobei innerhalb eines Moleküls R2 im Rahmen der gegebenen Definition unterschiedliche Bedeutungen haben kann.

Die erfindungsgemäß einzusetzenden ersten Tenside enthalten somit keine freien endständigen Hydroxylgruppen.

In einer bevorzugten Variante umfasst das erste Tensid endgruppenverschlossene Polyoxypropylenalkylether und/oder endgruppenverschlossene Polyoxyethlenpolyoxypropylenalkylether vorzugsweise in Mischung mit endgruppenverschlossenem Polyoxyethylenalkylether.

Bevorzugt ist das erste Tensid ein endgruppenverschlossenes Fettalkoholethoxylat. Fettalkoholethoxylate sind insbesondere nichtionische Tenside, die durch Umsetzung eines Fettalkohols (primärer Alkohol mit 6 bis 22 Kohlenstoffatomen) mit Ethylenoxid in Gegenwart basischer oder saurer Katalysatoren bei Temperaturen von 120 bis 200 °C und Drücken von 1 bis 10 bar gewonnen werden.

Ein bevorzugt eingesetztes erstes Tensid ist ein endgruppenverschlossenes Fettalkoholethoxylat mit einer Oberflächenspannung von 30 mN/m (in entionisiertem Wasser bei 20 C und einer Konzentration von 1 g/l) und einer Trübungstitrationszahl nach DIN 53989 von ca. 7.

Ein weiteres bevorzugt eingesetztes erstes Tensid ist ein endgruppenverschlossenes Fettalkoholethoxylat mit einer Oberflächenspannung von 29 mN/m (in entionisiertem Wasser bei 20 C und einer Konzentration von 1 g/l) und einer Trübungstitrationszahl nach DIN 53989 von ca. 22.

Ein weiteres bevorzugt eingesetztes erstes Tensid ist ein endgruppenverschlossenes Fettalkoholethoxylat mit einer Oberflächenspannung von 29 mN/m (in entionisiertem Wasser bei 20 C und einer Konzentration von 1 g/l) und einer Trübungstitrationszahl nach DIN 53989 von ca. 15.

Das erfindungsgemäß einzusetzende zweite Tensid ist ausgewählt aus der Gruppe der nichtionischen Fluortenside. Bevorzugt ist das nichtionische Fluortensid eine Verbindung der Formel

R_{f}-(CₙH₂ₙ)-Y-(CₘH₂ₘ-O)_{y}-R¹

worin R_{f} ein Rest der Formel CₓF₂ₓ₊₁ ist,
x eine ganze Zahl von 1 bis 30, vorzugsweise 2 bis 18 ist
n eine ganze Zahl von 0 bis 30, vorzugsweise 1 bis 3 ist,
Y -O- oder -CO-O- ist, vorzugsweise -O-,
m eine ganze Zahl von 2 bis 6, vorzugsweise 2 oder 3, besonders bevorzugt 2 ist,
y eine ganze Zahl von 1 bis 60, vorzugsweise 1 bis 25 ist und
R¹ Wasserstoff oder ein einwertiger organischer Rest, vorzugsweise Wasserstoff, C₁-C₆ Alkyl oder Phenyl ist, besonders bevorzugt Wasserstoff oder ein Alkylrest,
wobei m innerhalb eines Moleküls im Rahmen der gegebenen Definition unterschiedliche Werte annehmen kann.

Besonders bevorzugt ist das nichtionische Fluortensid eine Verbindung der Formel

R_{f}-(CH₂)₀₋₂₅-Y-(CH₂-CHR^{b}-O)₁₋₂₅-R¹

oder

R_{f}-(CH₂)₂-Y-(CH₂-CH₂-O)₁₋₂₅-R¹

worin R_{f} die oben definierte Bedeutung besitzt, und vorzugsweise ein Rest der Formel CₓF₂ₓ₊₁ mit x =1 bis 18, insbesondere x = 4 bis 12, ist,

Y -O- ist,

R^{b} C₁-C₆ Alkyl, vorzugsweise Methyl oder Ethyl ist, und
R₁ die oben definierte Bedeutung besitzt und vorzugsweise Wasserstoff, C₁-C₆ Alkyl, insbesondere Methyl, C₁-C₆ Alkenyl, vorzugsweise Vinyl, oder Phenyl ist.

Besonders bevorzugt ist das Fluortensid eine Verbindung der Formel

F-(CF₂-CF₂)₁₋₇-CH₂-CH₂-(O-CH₂-CH₂)₁₋₁₅-OH

oder

F-(CF₂-CF₂)₁₋₉-CH₂-CH₂-(O-CH₂-CH₂)₁₋₂₅-OH

Ein erfindungsgemäß besonders bevorzugt eingesetztes zweites Tensid ist ein ethoxyliertes nichtionisches Fluortensid mit einer Oberflächenspannung von 19 mN/m (in entionisiertem Wasser bei 25 °C und einer Konzentration von 0,1 g/l).

In einer erfindungsgemäßen Zusammensetzung, die ein Tensidsystem umfassend ein erstes, zweites und drittes Tensid enthält, ist das dritte Tensid ausgewählt aus der Gruppe der Silikontenside. Silikontenside sind Tenside, deren Moleküle mindestens eine ein Siliziumatom enthaltende Gruppe aufweisen. Bevorzugt sind nichtionische Silikontenside, insbesondere solche nichtionischen Silikontenside, die mindestens einen (Poly)-alkylenoxidrest aufweisen. Besonders bevorzugt sind nichtionische Tenside mit einer Molmasse von weniger als 6000 g/mol, die mindestens einen (Poly)alkylenoxidrest sowie eine Silizium enthaltende Gruppe aufweisen.

Ein erfindungsgemäß besonders bevorzugt eingesetztes drittes Tensid ist Polyalkylenoxid-modifiziertes Polydimethylsiloxan mit einer Oberflächenspannung von 20,5 mN/m (in entionisiertem Wasser bei 25 °C und einer Konzentration von 1 g/I).

Bevorzugt liegt die erfindungsgemäße Zusammensetzung in Form einer oder mehrerer Pasten vor.

Abhängig von der Auswahl der aushärtbaren Basiszusammensetzung kann die erfindungsgemäße Zusammensetzung als Einkomponenten- oder als Mehrkomponentensystem vorliegen. Im Falle eines Einkomponentensystems liegen alle Bestandteile der erfindungsgemäßen Zusammensetzung nebeneinander in einer Mischung vor. Im Falle eines Mehrkomponentensystems liegen die Bestandteile der erfindungsgemäßen Zusammensetzung in voneinander getrennten Komponenten vor, die erst unmittelbar vor dem Gebrauch miteinander vermischt werden. Wenn die Zusammensetzung ein mehrkomponentiges, vorzugsweise zweikomponentiges System umfassend eine erste Komponente und eine zweite Komponente ist, sind deren Bestandteile so aufeinander abgestimmt, dass durch Vermischen von erster und zweiter sowie der gegebenenfalls vorhandenen weiteren Komponenten die Aushärtung initiiert wird.

Wenn die erfindungsgemäße Zusammensetzung ein Zwei- oder Mehrkomponentensystem ist, liegen die zwei oder mehreren Komponenten bevorzugt in Form von Pasten vor.

Die aushärtbare Basiszusammensetzung der erfindungsgemäßen Zusammensetzung ist vorzugsweise ein aushärtbares Polymersystem. Die aushärtbare Basiszusammensetzung der erfindungsgemäßen Zusammensetzung ist vorzugsweise ausgewählt aus der Gruppe
- der durch Additionsreaktion vernetzenden Organopolysiloxane,
- der durch Kondensationsreaktion vernetzenden Organopolysiloxane,
- der durch Additionsreaktion vernetzenden Aziridinoreste enthaltenden Polyether,
- der durch Additionsreaktion vernetzenden Alkenylreste enthaltenden Polyether und
- der durch Kondensationsreaktion vernetzenden Alkoxysilylreste enthaltenden Polyether.

Bevorzugt ist die aushärtbare Basiszusammensetzung der erfindungsgemäßen Zusammensetzung ausgewählt aus der Gruppe der durch Additionsreaktion vernetzenden Organopolysiloxane.

Bevorzugt ist eine erfindungsgemäße Zusammensetzung (vorzugsweise ein Zwei- oder Mehrkomponentensystem) umfassend eine aushärtbare Basiszusammensetzung enthaltend ein durch Additionsreaktion vernetzendes Organopolysiloxan umfassend
- ein Organopolysiloxan mit mindestens zwei ethylenisch ungesättigten Gruppen
und
- ein Organohydrogenpolysiloxan.

Besonders bevorzugt ist die in der erfindungsgemäßen Zusammensetzung enthaltene aushärtbare Basiszusammensetzung ein durch Additionsreaktion vernetzendes Organopolysiloxan umfassend
- ein Organopolysiloxan mit mindestens zwei ethylenisch ungesättigten Gruppen
und
- ein Organohydrogenpolysiloxan.

Diese erfindungsgemäße aushärtbare Zusammensetzung ist bevorzugt ein Zwei- oder Mehrkomponentensystem, wobei
- eine erste Komponente Organopolysiloxan mit mindestens zwei ethylenisch ungesättigten Gruppen und das Organohydrogenpolysiloxan umfasst,
und
- eine zweite Komponente einen Katalysator, insbesondere einen Hydrosilylierungskatalysator umfasst,
wobei das erste Tensid und das zweite Tensid (sowie gegebenenfalls weitere Tenside) vorzugsweise in der ersten und/oder der zweiten Komponente enthalten sind.

Weiterhin bevorzugt ist eine erfindungsgemäße aushärtbare Zusammensetzung, die mehrere Komponenten, vorzugsweise zwei Komponenten umfassen, insbesondere
- eine erste Komponente umfassend das Organohydrogenpolysiloxan und optional Organopolysiloxan mit mindestens zwei ethylenisch ungesättigten Gruppen
und
- eine zweite Komponente umfassend Organopolysiloxan mit mindestens zwei ethylenisch ungesättigten Gruppen sowie einen Hydrosilylierungskatalysator
wobei das erste Tensid und das zweite Tensid (sowie gegebenenfalls weitere Tenside) vorzugsweise in der ersten und/oder der zweiten Komponente enthalten sind.

Bevorzugt sind die Organopolysiloxane mit mindestens zwei ethylenisch ungesättigten Gruppen ausgewählt aus der Gruppe bestehend aus
- Vinylpolydimethylsiloxan mit einer Viskosität von etwa 1000 mPas bei 20 °C
- Vinylpolydimethylsiloxan mit einer Viskosität von etwa 65000 mPas bei 20 °C

Bevorzugt sind die Organohydrogenpolysiloxane ausgewählt aus der Gruppe bestehend aus
- Dihydrogenpolydimethylsiloxan mit einem SiH-Gehalt von ca. 3,6 mmol/kg
- Polyhydrogenmethylsiloxan mit einem SiH-Gehalt von ca. 2,1 mmol/kg
- Polyhydrogenmethylsiloxan mit einem SiH-Gehalt von ca. 2,3 mmol/kg
- Polyhydrogenmethylsiloxan mit einem SiH-Gehalt von ca. 2,6 mmol/kg
- Polyhydrogenmethylsiloxan mit einem SiH-Gehalt von ca. 3.0 mmol/kg
- Polyhydrogenmethylsiloxan mit einem SiH-Gehalt von ca. 4,3 mmol/kg
- Polyhydrogenmethylsiloxan mit einem SiH-Gehalt von ca. 7,3 mmol/kg
- Polyhydrogenmethylsiloxan mit einem SiH-Gehalt von ca. 7,8 mmol/kg
- Polyhydrogenmethylsiloxan mit einem SiH-Gehalt von ca. 15 mmol/kg

Die erfindungsgemäße Zusammensetzung kann zudem einen oder mehrere Füllstoffe sowie gegebenenfalls weitere Additive umfassen. Diese sind dem Fachmann an sich bekannt. Beispielsweise kann die erfindungsgemäße Zusammensetzung Initiatoren oder Katalysatoren, die die Härtung initiieren bzw. beschleunigen, enthalten. Typischerweise werden Platin enthaltende Katalysatoren verwendet.

Bevorzugte Füllstoffe sind ausgewählt aus der Gruppe bestehend aus Aluminiumhydroxid, Zinkoxid, Titandioxid, Zirkoniumoxid, Siliciumdioxid (z.B. in Form von Quarzmehl oder gefällter und/oder pyrogener Kieselsaure). Vorzugsweise sind die Oberflächen der Partikel diese Füllstoffe mit organischen Resten modifiziert.

Eine erfindungsgemäße Zusammensetzung umfasst
- eine aushärtbare Basiszusammensetzung und
- ein Tensidsystem umfassend oder bestehend aus
   - 0,75 bis 3 Gew.-% eines ersten Tensids, welches auf in den Ansprüchen spezifizierte Weise ausgewählt ist aus der Gruppe bestehend aus endgruppenverschlossenem Polyoxyethylenalkylether, endgruppenverschlossenem Polyoxypropylenalkylether, endgruppen-verschlossenem Polyoxyethylenpolyoxypropylenalkylether und deren Mischungen,
   - 0,75 bis 3 Gew.-% eines zweiten Tensids, welches ausgewählt ist aus der Gruppe der nichtionischen Fluortenside,
   - 0 bis weniger als 0,5 Gew.-% eines dritten Tensids, welches ausgewählt ist aus der Gruppe der Silikontenside,
   - wobei die Gesamtmenge an erstem, zweitem und drittem Tensid im Bereich von 2 bis 6 Gew.-% liegt,
   - das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% beträgt,
wobei sämtliche Gewichtsprozentangaben bezogen sind auf die Gesamtmasse der Zusammensetzung.

Eine erfindungsgemäße Zusammensetzung umfasst
- eine aushärtbare Basiszusammensetzung und
- ein Tensidsystem umfassend oder bestehend aus
   - 0,5 bis 2,5 Gew.-% eines ersten Tensids, welches auf in den Ansprüchen spezifizierte Weise ausgewählt ist aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen,
   - 0,5 bis 2,5 Gew.-% eines zweiten Tensids, welches ausgewählt ist aus der Gruppe der nichtionischen Fluortenside,
   - 0,5 bis 2,5 Gew.-% eines dritten Tensids, welches ausgewählt ist aus der Gruppe der Silikontenside,
   wobei die Gesamtmenge an erstem, zweitem und drittem Tensid im Bereich von 1,75 Gew.-% bis 6 Gew.-% liegt
   und, wenn die Gesamtmenge an erstem, zweitem und drittem Tensid 2 Gew.-% oder weniger beträgt, die Menge des zweiten Tensids mindestens 0,75 Gew.-% beträgt,
   wobei sämtliche Gewichtsprozentangaben bezogen sind auf die Gesamtmasse der Zusammensetzung.

Mit den vorstehend beschriebenen Zusammensetzungen sind Abformmaterialien erhältlich, die bei der dentalen Abdrucknahme, also im noch nicht ausgehärteten Zustand nahezu spontan die feuchte, mit Speichel und gegebenenfalls Blut benetzte Zahnsubstanz und Mundschleimhaut benetzen und unmittelbar auf diese auffließen. Dies lässt sich z. B. für zwei- oder mehrkomponentige Systeme in Experimenten dadurch eindrucksvoll belegen, dass ein 40 Sekunden nach Beginn des Mischens der Komponenten des zwei- oder mehrkomponentigen aushärtbaren Basissystems an der Oberfläche des aushärtenden Abformmaterials applizierter Wassertropfen innerhalb von weniger als 10 Sekunden einen Kontaktwinkel von 10 ° erreicht. Ein Wassertropfen spreitet an den Abformmaterialien, die aus diesen beschriebenen Zusammensetzungen erhältlich sind, als nahezu spontan (initial).

Die Erfindung umfasst, wie vorstehend beschrieben, eine aushärtbare Zusammensetzung mit einem Tensidsystem, das neben einem ersten Tensid wie oben beschrieben und einem zweiten Tensid wie oben beschrieben als drittes Tensid ein Tensid ausgewählt aus der Gruppe der Silikontenside. Es ist jedoch für manche Zwecke bevorzugt, dass die erfindungsgemäße Zusammensetzung frei ist von nichtionischen Tensiden mit einer Molmasse von weniger als 6000 g/mol, speziell weniger als 4000 g/mol, und insbesondere von 350 bis 2000 g/mol, die mindestens einen (Poly)alkylenoxidrest sowie eine Silizium enthaltende Gruppe aufweisen. Insbesondere ist die erfindungsgemäße Zusammensetzung vorzugsweise frei von
- den in der oben genannten Patentanmeldung DE 10 2006 001 126 A1 offenbarten Organosiloxantensiden der Formel II und/oder der Formel III sowie
- den in der oben genannten Patentanmeldung DE 10 2006 001 126 A1 offenbarten Organocarbonsilantensiden der Formel IV, V und VI und
- den in der oben genannten Patentanmeldung DE 10 2006 001 126 A1 offenbarten Silizium und mindestens einen (Poly)alkylenoxidrest aufweisenden nichtionischen Tensiden der Formel VII, VIII, IX und X.

Ebenfalls bevorzugt ist eine erfindungsgemäße Zusammensetzung, die frei ist von
- den in der oben genannten Patentanmeldung DE 10 2009 021 553 A1 offenbarten Organosilantensiden der Formel II und der Formel III sowie den in der oben genannten Patentanmeldung DE 10 2009 021 553 A1 offenbarten Organocarbonsilantensiden der Formeln IV, V und VI sowie den in der oben genannten Patentanmeldung DE 10 2009 021 553 A1 offenbarten Silizium und mindestens einen (Poly)alkylenoxidrest aufweisenden nichtionischen Tensiden der Formeln VII, VIII, IX und X
   und/oder
- den in der oben genannten Patentanmeldung DE 10 2009 021 553 A1 offenbarten Fluortensiden a) bis h).

Besonders bevorzugt ist eine erfindungsgemäße Zusammensetzung, die frei von nichtionisches Tensiden ist, die mindestens einen (Poly)alkylenoxidrest sowie eine Silizium enthaltende Gruppe aufweisen. Eine ganz besonders bevorzugte erfindungsgemäße Zusammensetzung ist insgesamt frei von Silizium aufweisenden Tensiden.

Ebenfalls bevorzugt ist eine erfindungsgemäße Zusammensetzung, die frei ist von
- den in der oben genannten Patentanmeldung WO 2009/079534 A2, Ansprüche 1 bis 11, definierten Fluor enthaltenden Verbindungen
und/oder
- den in der oben genannten Patentanmeldung WO 2009/079534 A2, Anspruch 12, definierten Tensiden
und/oder
- Polyethylenglycoldimethylether.

Weiter bevorzugt ist eine erfindungsgemäße Zusammensetzung, die frei ist von
(i) den in der oben genannten Patentanmeldung DE 10 2006 001 126 A1 offenbarten Organosiloxantensiden der Formel II und der Formel III sowie
   den in der oben genannten Patentanmeldung DE 10 2006 001 126 A1 offenbarten Organocarbonsilantensiden der Formel IV, V und VI und
   den in der oben genannten Patentanmeldung DE 10 2006 001 126 A1 offenbarten Silizium und mindestens einen (Poly)alkylenoxidrest aufweisenden nichtionischen Tensiden der Formeln VII, VIII, IX und X;
   sowie
(ii) den in der oben genannten Patentanmeldung DE 10 2009 021 553 A1 offenbarten Organosilantensiden der Formel II und der Formel III sowie den in der oben genannten Patentanmeldung DE 10 2009 021 553 A1 offenbarten Organocarbonsilantensiden der Formeln IV, V und VI sowie den in der oben genannten Patentanmeldung DE 10 2009 021 553 A1 offenbarten Silizium und mindestens einen (Poly)alkylenoxidrest aufweisenden nichtionischen Tensiden der Formeln VII, VIII, IX und X
   und/oder
   den in der oben genannten Patentanmeldung DE 10 2009 021 553 A1 offenbarten Fluortensiden a) bis h);
   sowie
(iii) den in der oben genannten Patentanmeldung WO 2009/079534 A2, Ansprüche 1 bis 11, definierten Fluor enthaltenden Verbindungen
   und/oder
   den in der oben genannten Patentanmeldung WO 2009/079534 A2, Anspruch 12, definierten Tensiden
   und/oder
   Polyethylenglycoldimethylether.

Besonders bevorzugt ist eine erfindungsgemäße Zusammensetzung, die frei ist von sämtlichen Verbindungen, von denen vorstehend erwähnt wurde, dass eine erfindungsgemäße Zusammensetzung vorzugsweise frei von der jeweiligen Verbindung ist.

Die Erfindung betrifft außerdem die Verwendung einer erfindungsgemäßen Zusammensetzung als dentales Abformsystem. Unter einem dentalen Abformsystem wird eine ein- oder mehrkomponentige Zusammensetzung verstanden, die - im Falle einer mehrkomponentigen Zusammensetzung unmittelbar nach dem Mischen der Komponenten - zur Herstellung eines dentalen Abdrucks eines Patienten eingesetzt wird und dabei aushärtet.

Beschrieben wird auch ein Verfahren zur Herstellung einer erfindungsgemäßen Zusammensetzung, umfassend die Schritte:
- Bereitstellen einer aushärtbaren Basiszusammensetzung
   - Bereitstellen eines Tensidsystems umfassend oder bestehend aus
   - 0,5 bis 3 Gew.-%, eines ersten Tensids, welches ausgewählt ist aus der Gruppe bestehend aus endgruppenverschlossenem Polyoxyethylenalkylether, endgruppenverschlossenem Polyoxypropylenalkylether, endgruppenverschlossenem Polyoxyethylenpolyoxypropylenalkylether und deren Mischungen,
   - 0,5 bis 3 Gew.-% eines zweiten Tensids, welches ausgewählt ist aus der Gruppe der nichtionischen Fluortenside,
   - 0 bis 3 Gew.-% eines dritten Tensids, welches ausgewählt ist aus der Gruppe der Silikontenside,
   - wobei die Gesamtmenge an erstem, zweitem und drittem Tensid im Bereich von 1,5 Gew.-% bis 6 Gew.-% liegt,
   mit der Maßgabe, dass
   - das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% oder weniger beträgt,
   wobei sämtliche Gewichtsprozentangaben bezogen sind auf die Gesamtmasse der Zusammensetzung.

Eine mit dem beschriebenen Verfahren hergestellte Zusammensetzung ist bevorzugt ein mehrkomponentiges, vorzugsweise zweikomponentiges System umfassend eine erste Komponente und eine zweite Komponente, deren Bestandteile so aufeinander abgestimmt sind, dass durch Vermischen von erster und zweiter sowie der gegebenenfalls vorhandenen weiteren Komponenten die Aushärtung initiiert wird, wobei das erste Tensid und das zweite Tensid (sowie gegebenenfalls weitere Tenside) vorzugsweise in der ersten und/oder der zweiten Komponente enthalten sind.

Die Erfindung wird nachfolgend anhand von Beispielen näher erläutert.

### Ausführungsbeispiele:

Alle Ausführungsbeispiele beziehen sich auf zweikomponentige Zusammensetzungen mit einer aushärtbaren Basiszusammensetzung ausgewählt aus der Gruppe der durch Additionsreaktion vernetzenden Organopolysiloxane. Die Zusammensetzung umfasst die Komponenten I und II. Komponente I umfasst die Basismasse der aushärtbaren Basiszusammensetzung und enthält Organohydrogenpolysiloxane (Siloxane 3 und 4, s.u.) und ein Organopolysiloxan mit mindestens zwei ethylenisch ungesättigten Gruppen (Siloxan 1) sowie gegebenenfalls ein oder mehrere Tenside. Komponente II umfasst die Katalysatorkomponente der aushärtbaren Basiszusammensetzung und enthält Organopolysiloxane mit mindestens zwei ethylenisch ungesättigten Gruppen (Siloxane 1 und 2, s.u.), einen Hydrosilylierungskatalysator sowie gegebenenfalls ein oder mehrere Tenside.

Für die Herstellung der Komponenten I und II wurden folgende Substanzen eingesetzt:
- Siloxan 1: Vinylpolydimethylsiloxan mit einer Viskosität von ca. 1000 mPas bei 20 °C
- Siloxan 2: Vinylpolydimethylsiloxan mit einer Viskosität von ca. 65000 mPas bei 20 °C
- Siloxan 3: Dihydrogenpolydimethylsiloxan mit einem SiH-Gehalt von ca. 3,6 mmol/kg
- Siloxan 4: Polyhydrogenmethylsiloxan mit einem SiH-Gehalt von ca. 2,3 mmol/kg
- Quarzmehl 1: Hydrophobisiertes Quarzmehl mit einer mittleren Korngröße von 3 µm
- Katalysator 1: Platin-Katalysator mit einem Platin-Gehalt von 1 Gew,-%
- Tensid 1: Endgruppenverschlossenes Fettalkoholethoxylat mit einer Oberflächenspannung von 30 mN/m (in entionisiertem Wasser bei 20 °C und einer Konzentration von 1 g/l)
- Tensid 2: Ethoxyliertes nichtionisches Fluortensid mit einer Oberflächenspannung von 19 mN/cm (in entionisiertem Wasser bei 25 °C und einer Konzentration von 0,1 g/l)
- Tensid 3: Polyalkylenoxid-modifiziertes Polydimethylsiloxan mit einer Oberflächenspannung von 20.5 mN/m (in entionisiertem Wasser bei 25 °C und einer Konzentration von 1 g/l)

Für die Ausführungsbeispiele wurden folgende Varianten der Komponente I hergestellt:
Komponente I Grundrezeptur (ohne Tenside)

In einem Taumelmischer wurden 41 Teile eines Siloxans 1, 12 Teile eines Siloxans 4, 2 Teile eines Siloxans 3 und 45 Teile eines Quarzmehls 1 homogen gemischt. Anschließend wurde 20 Minuten im Vakuum entgast.

### Komponente I Variante 1

99,5 Teile der Grundrezeptur wurden mit 0,5 Teilen Tensid 1 in einem Taumelmischer homogen gemischt und im Vakuum 20 Minuten entgast.

### Komponente I Variante 2

99 Teile der Grundrezeptur wurden mit 1 Teil Tensid 1 in einem Taumelmischer homogen gemischt und im Vakuum 20 Minuten entgast.

### Komponente I Variante 3

98 Teile der Grundrezeptur wurden mit 2 Teilen Tensid 1 in einem Taumelmischer homogen gemischt und im Vakuum 20 Minuten entgast.

### Komponente I Variante 4

99 Teile der Grundrezeptur wurden mit 1 Teil Tensid 3 in einem Taumelmischer homogen gemischt und im Vakuum 20 Minuten entgast.

### Komponente I Variante 5

98 Teile der Grundrezeptur wurden mit 2 Teilen Tensid 3 in einem Taumelmischer homogen gemischt und im Vakuum 20 Minuten entgast.

### Komponente I Variante 6

98 Teile der Grundrezeptur wurden mit 1 Teil Tensid 1 und mit 1 Teil Tensid 2 in einem Taumelmischer homogen gemischt und im Vakuum 20 Minuten entgast.

### Komponente I Variante 7

97,5 Teile der Grundrezeptur wurden mit 1 Teil Tensid 1 und mit 1,5 Teilen Tensid 2 in einem Taumelmischer homogen gemischt und im Vakuum 20 Minuten entgast.

### Komponente I Variante 8

96,5 Teile der Grundrezeptur wurden mit 1 Teil Tensid 1 und mit 2,5 Teilen Tensid 2 in einem Taumelmischer homogen gemischt und im Vakuum 20 Minuten entgast.

Für die Ausführungsbeispiele wurden folgende Varianten der Komponente II hergestellt:

### Komponente II Variante A

In einem Taumelmischer wurden 43 Teile eines Siloxans 1, 11 Teile eines Siloxans 2, 45 Teile eines Quarzmehls 1 und 1 Teil eines Katalysators 1 homogen gemischt. Anschließend wurde 20 Minuten im Vakuum entgast.

### Komponente II Variante B

99,5 Teile der Grundrezeptur der Komponente II gemäß Variante A wurden mit 0,5 Teilen Tensid 1 in einem Taumelmischer homogen gemischt und im Vakuum 20 Minuten entgast.

### Komponente II Variante C

99 Teile der Grundrezeptur der Komponente II gemäß Variante A wurden mit 1 Teil Tensid 1 in einem Taumelmischer homogen gemischt und im Vakuum 20 Minuten entgast.

### Komponente II Variante D

98 Teile der Grundrezeptur der Komponente II gemäß Variante A wurden mit 2 Teilen Tensid 1 in einem Taumelmischer homogen gemischt und im Vakuum 20 Minuten entgast.

### Komponente II Variante E

99 Teile der Grundrezeptur der Komponente II gemäß Variante A wurden mit 1 Teil Tensid 3 in einem Taumelmischer homogen gemischt und im Vakuum 20 Minuten entgast.

### Komponente II Variante F

98 Teile der Grundrezeptur der Komponente II gemäß Variante A wurden mit 2 Teilen Tensid 3 in einem Taumelmischer homogen gemischt und im Vakuum 20 Minuten entgast.

### Komponente II Variante G

95 Teile der Grundrezeptur der Komponente II gemäß Variante A wurden mit 5 Teilen Tensid 3 in einem Taumelmischer homogen gemischt und im Vakuum 20 Minuten entgast.

Durch Kombination der verschiedenen Varianten der Komponenten I und II wurden die in den nachfolgenden erfindungsgemäßen Beispielen und nicht erfindungsgemäßen Vergleichsbeispielen beschriebenen Zusammensetzungen hergestellt. Die Nomenklatur der Beispiele bzw. Vergleichsbeispiele spiegelt wieder, welche Variante (A-G) der Komponente II mit welcher Variante der Komponente I (1-7) kombiniert wurde. Eine Kombination von Variante A der Komponente II mit Variante 1 der Komponente I bildet somit das Beispiel A1.

Herstellung und Prüfung der Abformmaterialien erfolgte nach ISO 4823.

Zur Bestimmung der Kontaktwinkel wurden dynamische Kontaktwinkelmessungen mit dem Kontaktwinkelmessgerät DSA100 (Fa. Krüss, Hamburg) durchgeführt, welches mit einem vollautomatischen Messsystem und einer Temperierkammer für die Proben ausgestattet war. 40 Sekunden nach Beginn des Mischens der Komponenten I und II wurde ein Wassertropfen von 5 µl Volumen auf der Oberfläche des aushärtenden Materials plaziert. Die Kontaktwinkel wurden dynamisch für 200 Sekunden mittels der Methode "circle fit" bestimmt.

In Tabelle 1 sind die Kontaktwinkel angegeben, die nach 30 Sekunden erhalten wurde. Mit sämtlichen erfindungsgemäßen Beispielen wurde ein Kontaktwinkel von 10 ° erreicht. Die beim Aushärten dieser Zusammensetzungen entstehenden dentalen Abformmaterialien sind somit hinreichend hydrophil, um schnell auf die feuchte, mit Speichel und ggf. Blut bedeckte Zahnsubstanz und Mundschleimhaut aufzufließen, so dass ein detailgetreuer Abdruck der dentalen Situation erhalten wird.

In Tabelle 2 sind für die erfindungsgemäßen Beispiele und für Vergleichsbeispiele die Zeiten angegeben, innerhalb derer sich der Kontaktwinkel von 10° einstellt. Zusammensetzungen, bei denen sich der Kontaktwinkel von 10° in einer Zeit von weniger als 10 Sekunden einstellt, sind erfindungsgemäß bevorzugt.

### Zusammensetzungen umfassend Tensid 1 und Tensid 2

### Beispiel A8 (Vergleichsbeispiel):

50 Teile der Paste der Komponente II gemäß Variante A wurden mit 50 Teilen Paste der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 21 Sekunden einen Kontaktwinkel kleiner 10 ° erreichte.

### Beispiel B7 (Vergleichsbeispiel):

50 Teile der Paste der Komponente II gemäß Variante B wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel kleiner 10 ° erreichte.

### Beispiel B8 (erfindungsgemäßes Beispiel):

50 Teile der Paste der Komponente II gemäß Variante B wurden mit 50 Teilen Paste der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel kleiner 10 ° erreichte.

### Beispiel C7 (Vergleichsbeispiel):

50 Teile der Paste der Komponente II gemäß Variante C wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel kleiner 10 ° erreichte.

### Beispiel C8 (erfindungsgemäßes Beispiel):

50 Teile der Paste der Komponente II gemäß Variante C wurden mit 50 Teilen Paste aus der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel kleiner 10 ° erreichte.

### Beispiel D7 (erfindungsgemäßes Beispiel):

50 Teile der Paste der Komponente II gemäß Variante D wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel kleiner 10 ° erreichte.

### Beispiel D8 (erfindungsgemäßes Beispiel):

50 Teile der Paste der Komponente II gemäß Variante D wurden mit 50 Teilen Paste aus der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel kleiner 10 ° erreichte.

### Zusammensetzungen umfassend Tensid 3, Tensid 1 und Tensid 2

### Beispiel E6 (Vergleichsbeispiel):

50 Teile der Paste der Komponente II gemäß Variante E wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 28 Sekunden einen Kontaktwinkel kleiner 10 ° erreichte.

### Beispiel E7 (Vergleichsbeispiel):

50 Teile der Paste der Komponente II gemäß Variante E wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel kleiner 10 ° erreichte.

### Beispiel E8 (Vergleichsbeispiel):

50 Teile der Paste der Komponente II gemäß Variante E wurden mit 50 Teilen Paste aus der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Beispiel F6 (Vergleichsbeispiel):

50 Teile der Paste der Komponente II gemäß Variante F wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 12 Sekunden einen Kontaktwinkel von < 10 ° erreichte.

### Beispiel F7 (Vergleichsbeispiel):

50 Teile der Paste der Komponente II gemäß Variante F wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Beispiel F8 (Vergleichsbeispiel):

50 Teile der Paste der Komponente II gemäß Variante F wurden mit 50 Teilen Paste aus der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Beispiel G6 (Vergleichsbeispiel):

50 Teile der Paste der Komponente II gemäß Variante G wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Beispiel G7 (Vergleichsbeispiel):

50 Teile der Paste der Komponente II gemäß Variante G wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Beispiel G8 (Vergleichsbeispiel):

50 Teile der Paste der Komponente II gemäß Variante G wurden mit 50 Teilen Paste aus der Komponente I gemäß Variante 8 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen innerhalb von 10 Sekunden einen Kontaktwinkel von < 10 ° erreichte.

### Vergleichsbeispiele (nicht erfindungsgemäß)

### Vergleichsbeispiele enthaltend nur Tensid 1

### Beispiel A1 (Vergleichsbeispiel mit 0,25% Tensid 1):

50 Teile der Paste der Komponente II gemäß Variante A wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel > 90 ° aufwies.

### Beispiel A2 (Vergleichsbeispiel mit 0,5% Tensid 1):

50 Teile der Paste der Komponente II gemäß Variante A wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel > 90 ° aufwies.

### Beispiel A3 (Vergleichsbeispiel mit 1% Tensid 1):

50 Teile der Paste der Komponente II gemäß Variante A wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 70 ° erreichte.

### Beispiel B1 (Vergleichsbeispiel mit 0,5% Tensid 1):

50 Teile der Paste der Komponente II gemäß Variante B wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel > 90 ° aufwies.

### Beispiel B2 (Vergleichsbeispiel mit 0,75% Tensid 1):

50 Teile der Paste der Komponente II gemäß Variante B wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel > 90 ° aufwies.

### Beispiel B3 (Vergleichsbeispiel mit 1,25% Tensid 1):

50 Teile der Paste der Komponente II gemäß Variante B wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 58 ° aufwies, ein Kontaktwinkel kleiner 10 ° wurde nicht erreicht.

### Beispiel C1 (Vergleichsbeispiel mit 0,75% Tensid 1):

50 Teile der Paste der Komponente II gemäß Variante C wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel > 90 ° aufwies.

### Beispiel C2 (Vergleichsbeispiel mit 1% Tensid 1):

50 Teile der Paste der Komponente II gemäß Variante C wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 64 ° aufwies, ein Kontaktwinkel kleiner 10 ° wurde nicht erreicht.

### Beispiel C3 (Vergleichsbeispiel mit 1,5% Tensid 1):

50 Teile der Paste der Komponente II gemäß Variante C mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 53 ° aufwies, ein Kontaktwinkel kleiner 10 ° wurde nicht erreicht.

### Beispiel D1 (Vergleichsbeispiel mit 1,25% Tensid 1):

50 Teile der Paste der Komponente II gemäß Variante D wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 56 ° aufwies, ein Kontaktwinkel kleiner 10 ° wurde nicht erreicht.

### Beispiel D2 (Vergleichsbeispiel mit 1,5% Tensid 1):

50 Teile der Paste der Komponente II gemäß Variante D wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 52 ° aufwies, ein Kontaktwinkel kleiner 10 ° wurde nicht erreicht.

### Beispiel D3 (Vergleichsbeispiel mit 2% Tensid 1):

50 Teile der Paste der Komponente II gemäß Variante D wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 50 ° aufwies, ein Kontaktwinkel kleiner 10 ° wurde nicht erreicht.

### Vergleichsbeispiele enthaltend nur Tensid 3

### Beispiel A4 (Vergleichsbeispiel mit 0,5% Tensid 3):

50 Teile der Paste der Komponente II gemäß Variante A wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 64 ° erreichte.

### Beispiel A5 (Vergleichsbeispiel mit 1% Tensid 3):

50 Teile der Paste der Komponente II gemäß Variante A wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 55 ° erreichte.

### Beispiel E4 (Vergleichsbeispiel mit 1% Tensid 3):

50 Teile der Paste der Komponente II gemäß Variante E wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 53 ° erreichte.

### Beispiel E5 (Vergleichsbeispiel mit 1,5% Tensid 3):

50 Teile der Paste der Komponente II gemäß Variante E wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 30 ° erreichte.

### Beispiel F4 (Vergleichsbeispiel mit 1,5% Tensid 3):

50 Teile der Paste der Komponente II gemäß Variante F wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 32 ° erreichte.

### Beispiel F5 (Vergleichsbeispiel mit 2% Tensid 3):

50 Teile der Paste der Komponente II gemäß Variante F wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 19 ° erreichte.

### Beispiel G4 (Vergleichsbeispiel mit 3% Tensid 3):

50 Teile der Paste der Komponente II gemäß Variante G wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 17 ° erreichte.

### Beispiel G5 (Vergleichsbeispiel mit 3,5% Tensid 3):

50 Teile der Paste der Komponente II gemäß Variante G wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 15 ° erreichte.

### Vergleichsbeispiele enthaltend Tensid 3 und Tensid 1

### Beispiel B4 (Vergleichsbeispiel mit 0,5% Tensid 3 und 0,25 % Tensid 1):

50 Teile der Paste der Komponente II gemäß Variante B wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 62 ° aufwies, ein Kontaktwinkel kleiner 10 ° wurde nicht erreicht.

### Beispiel B5 (Vergleichsbeispiel mit 1% Tensid 3 und 0,25 % Tensid 1):

50 Teile der Paste der Komponente II gemäß Variante B wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 45 ° aufwies, ein Kontaktwinkel kleiner 10 ° wurde nicht erreicht.

### Beispiel C4 (Vergleichsbeispiel mit 0,5% Tensid 3 und 0,5 % Tensid 1):

50 Teile der Paste der Komponente II gemäß Variante C wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 47 ° aufwies, ein Kontaktwinkel kleiner 10 ° wurde nicht erreicht.

### Beispiel C5 (Vergleichsbeispiel mit 1% Tensid 3 und 0,5 % Tensid 1):

50 Teile der Paste der Komponente II gemäß Variante C wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 40 ° aufwies, ein Kontaktwinkel kleiner 10 ° wurde nicht erreicht.

### Beispiel D4 (Vergleichsbeispiel mit 0,5% Tensid 3 und 1 % Tensid 1):

50 Teile der Paste der Komponente II gemäß Variante D wurden mit 50 Teilen Paste der Komponente I gemäß Variante 4 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 31 ° aufwies, ein Kontaktwinkel kleiner 10 ° wurde nicht erreicht.

### Beispiel D5 (Vergleichsbeispiel mit 1% Tensid 3 und 1 % Tensid 1):

50 Teile der Paste der Komponente II gemäß Variante D wurden mit 50 Teilen Paste der Komponente I gemäß Variante 5 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 22 ° aufwies, ein Kontaktwinkel kleiner 10 ° wurde nicht erreicht.

### Beispiel E1:

50 Teile der Paste der Komponente II gemäß Variante E wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 67 ° aufwies, ein Kontaktwinkel unter 10 ° wurde nicht erreicht.

### Beispiel E2:

50 Teile der Paste der Komponente II gemäß Variante E wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 61 ° aufwies, ein Kontaktwinkel unter 10 ° wurde nicht erreicht.

### Beispiel E3:

50 Teile der Paste der Komponente II gemäß Variante E wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 39 ° aufwies, ein Kontaktwinkel unter 10 ° wurde nicht erreicht.

### Beispiel F1:

50 Teile der Paste der Komponente II gemäß Variante F wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 47 ° aufwies, ein Kontaktwinkel unter 10 ° wurde nicht erreicht.

### Beispiel F2:

50 Teile der Paste der Komponente II gemäß Variante F wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 40 ° aufwies, ein Kontaktwinkel unter 10 ° wurde nicht erreicht.

### Beispiel F3:

50 Teile der Paste der Komponente II gemäß Variante F wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 39 ° aufwies, ein Kontaktwinkel unter 10 ° wurde nicht erreicht.

### Beispiel G1:

50 Teile der Paste der Komponente II gemäß Variante G wurden mit 50 Teilen Paste der Komponente I gemäß Variante 1 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 18 ° aufwies, ein Kontaktwinkel unter 10 ° wurde nicht erreicht.

### Beispiel G2:

50 Teile der Paste der Komponente II gemäß Variante G wurden mit 50 Teilen Paste der Komponente I gemäß Variante 2 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 20 ° aufwies, ein Kontaktwinkel unter 10 ° wurde nicht erreicht.

### Beispiel G3:

50 Teile der Paste der Komponente II gemäß Variante G wurden mit 50 Teilen Paste der Komponente I gemäß Variante 3 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 21 ° aufwies, ein Kontaktwinkel unter 10 ° wurde nicht erreicht.

### Vergleichsbeispiele enthaltend Tensid 1 und Tensid 2

### Beispiel A6:

50 Teile der Paste der Komponente II gemäß Variante A wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 85 ° aufwies.

### Beispiel A7:

50 Teile der Paste der Komponente II gemäß Variante A wurden mit 50 Teilen Paste der Komponente I gemäß Variante 7 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 20° aufwies, ein Kontaktwinkel von < 10 °wurde nach 44 Sekunden erreicht.

### Beispiel B6:

50 Teile der Paste der Komponente II gemäß Variante B wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 45 ° hatte, ein Kontaktwinkel kleiner 10 ° wurde nach 80 Sekunden erreicht.

### Beispiel C6:

50 Teile der Paste der Komponente II gemäß Variante C mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 25 ° hatte, ein Kontaktwinkel kleiner 10 ° wurde nach 65 Sekunden erreicht.

### Beispiel D6:

50 Teile der Paste der Komponente II gemäß Variante D wurden mit 50 Teilen Paste der Komponente I gemäß Variante 6 in eine Kartusche (Fa. Mixpac) abgefüllt und über einen statischen Mischer als homogene Mischung ausgedrückt.

Es wurde ein dünnfließendes Abformmaterial nach ISO 4823 erhalten, auf welchem ein aufgebrachter Wassertropfen nach 30 Sekunden einen Kontaktwinkel von 13 ° hatte, ein Kontaktwinkel kleiner 10 ° wurde nach 39 Sekunden erreicht.

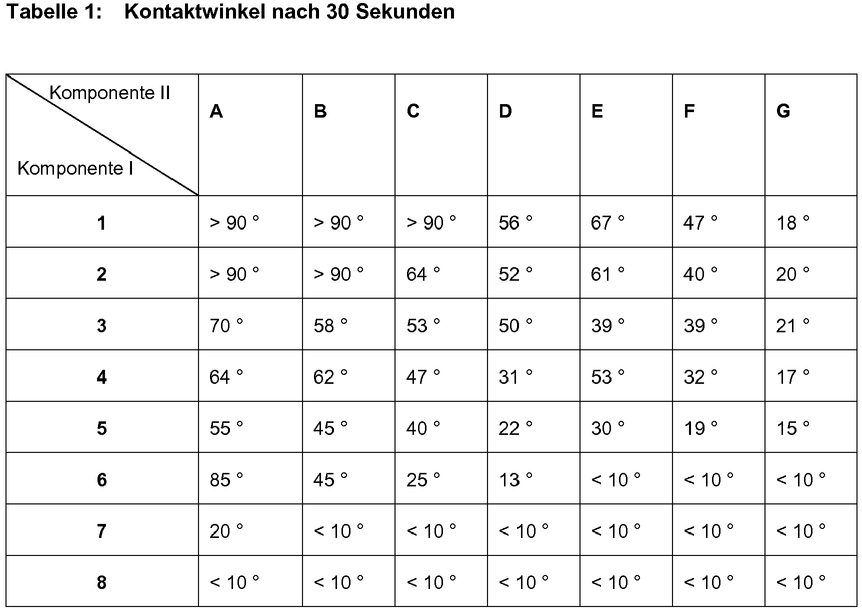

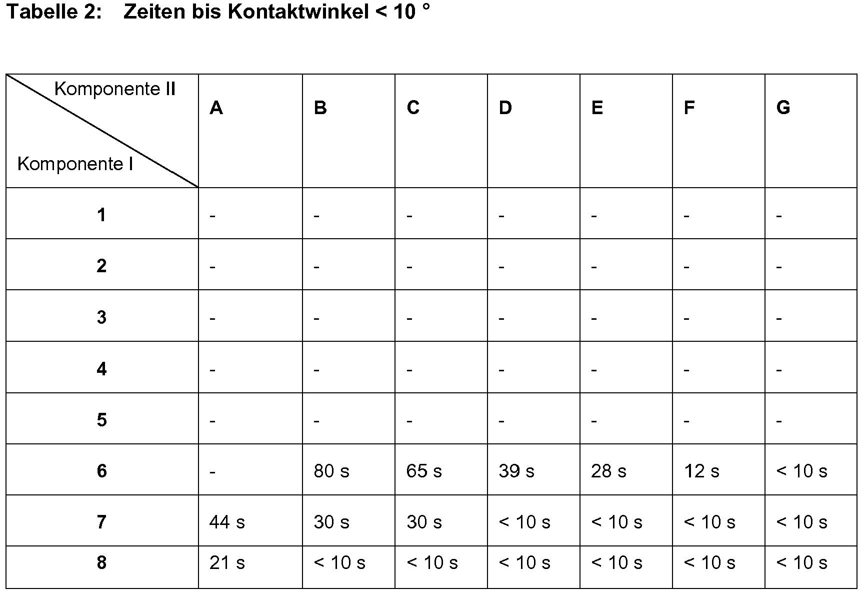

## Patentansprüche

1. Aushärtbare, ein- oder mehrkomponentige Zusammensetzung zur dentalen Abformung, umfassend
- eine aushärtbare Basiszusammensetzung
und
- ein Tensidsystem umfassend oder bestehend aus
- 0,75 bis 3 Gew.-% eines ersten Tensids der Formel 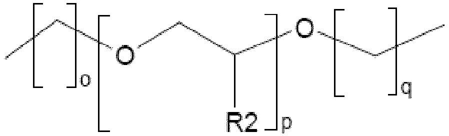 worin
o eine ganze Zahl von 5 bis 22 ist,
p eine ganze Zahl von 2 bis 20 ist, und
q eine ganze Zahl von 0 bis 5 ist,
R2 Wasserstoff oder eine Methylgruppe ist, wobei innerhalb eines Moleküls R2 im Rahmen der gegebenen Definition unterschiedliche Bedeutungen haben kann,
- 0,75 bis 3 Gew.-% eines zweiten Tensids, welches ausgewählt ist aus der Gruppe der nichtionischen Fluortenside,
- 0 bis weniger als 0,5 Gew.-% eines dritten Tensids, welches ausgewählt ist aus der Gruppe der Silikontenside,
- wobei die Gesamtmenge an erstem, zweitem und drittem Tensid im Bereich von 2 bis 6 Gew.-% liegt,
mit der Maßgabe, dass
- das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% oder weniger beträgt,
wobei sämtliche Gewichtsprozentangaben bezogen sind auf die Gesamtmasse der Zusammensetzung.

2. Aushärtbare Zusammensetzung nach Anspruch 1, wobei die Zusammensetzung ein mehrkomponentiges, vorzugsweise zweikomponentiges System ist, umfassend eine erste Komponente und eine zweite Komponente, deren Bestandteile so aufeinander abgestimmt sind, dass durch Vermischen von erster und zweiter sowie der gegebenenfalls vorhandenen weiteren Komponenten die Aushärtung initiiert wird.

3. Aushärtbare Zusammensetzung nach einem der vorhergehenden Ansprüche, wobei die aushärtbare Basiszusammensetzung ausgewählt ist aus der Gruppe
- der durch Additionsreaktion vernetzenden Organopolysiloxane,
- der durch Kondensationsreaktion vernetzenden Organopolysiloxane,
- der durch Additionsreaktion vernetzenden Aziridinoreste enthaltenden Polyether,
- der durch Additionsreaktion vernetzenden Alkenylreste enthaltenden Polyether und
- der durch Kondensationsreaktion vernetzenden Alkoxysilylreste enthaltenden Polyether.

4. Aushärtbare Zusammensetzung nach Anspruch 3, wobei die aushärtbare Basiszusammensetzung ein durch Additionsreaktion vernetzendes Organopolysiloxan ist ist umfassend
- ein Organopolysiloxan mit mindestens zwei ethylenisch ungesättigten Gruppen
und
- ein Organohydrogenpolysiloxan.

5. Aushärtbare Zusammensetzung nach Anspruch 4, wobei die Zusammensetzung ein mehrkomponentiges, vorzugsweise zweikomponentiges System ist, umfassend
- eine erste Komponente umfassend Organopolysiloxan mit mindestens zwei ethylenisch ungesättigten Gruppen sowie einen Hydrosilylierungskatalysator
und
- eine zweite Komponente umfassend das Organohydrogenpolysiloxan und optional Organopolysiloxan mit mindestens zwei ethylenisch ungesättigten Gruppen,
wobei das erste Tensid und das zweite Tensid (sowie gegebenenfalls weitere Tenside) vorzugsweise in der ersten und/oder der zweiten Komponente enthalten sind.

6. Aushärtbare Zusammensetzung nach einem der vorhergehenden Ansprüche, weiter umfassend ein oder mehrere Füllstoffe sowie gegebenenfalls weitere Additive.

7. Aushärtbare Zusammensetzung nach einem der vorhergehenden Ansprüche, wobei das zweite Tensid eine Verbindung der Formel
R_{f}-(CₙH₂ₙ)-Y-(CₘH₂ₘ-O)_{y}-R¹
ist, worin
- R_{f} ein Rest der Formel CₓF₂ₓ₊₁ ist,
- x eine ganze Zahl von 1 bis 30, vorzugsweise 2 bis 18 ist,
- n eine ganze Zahl von 0 bis 30, vorzugsweise 1 bis 3 ist,
- Y -O- oder -CO-O- ist, vorzugsweise -O-,
- m eine ganze Zahl von 2 bis 6, vorzugsweise 2 oder 3, besonders bevorzugt 2 ist, wobei m innerhalb eines Moleküls im Rahmen der gegebenen Definition unterschiedliche Werte annehmen kann,
- y eine ganze Zahl von 1 bis 60, vorzugsweise 1 bis 25 ist und
- R¹ Wasserstoff oder ein einwertiger organischer Rest ist, vorzugsweise Wasserstoff, C₁-C₆ Alkyl oder Phenyl ist, besonders bevorzugt Wasserstoff oder ein Alkylrest.

8. Verwendung einer Zusammensetzung nach einem der vorhergehenden Ansprüche als dentales Abformsystem.

9. Verfahren zur Herstellung einer Zusammensetzung nach einem der Ansprüche 1 bis 7, umfassend die Schritte:
- Bereitstellen einer aushärtbaren Basiszusammensetzung,
- Bereitstellen eines Tensidsystems umfassend oder bestehend aus
- 0,75 bis 3 Gew.-% eines ersten Tensids der Formel 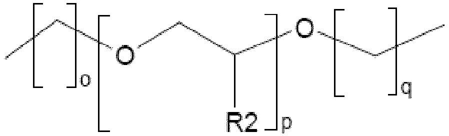 worin
o eine ganze Zahl von 5 bis 22 ist,
p eine ganze Zahl von 2 bis 20 ist, und
q eine ganze Zahl von 0 bis 5 ist,
R2 Wasserstoff oder eine Methylgruppe ist, wobei innerhalb eines Moleküls R2 im Rahmen der gegebenen Definition unterschiedliche Bedeutungen haben kann,
- 0,75 bis 3 Gew.-% eines zweiten Tensids, welches ausgewählt ist aus der Gruppe der nichtionischen Fluortenside,
- 0 bis weniger als 0,5 Gew.-% eines dritten Tensids, welches ausgewählt ist aus der Gruppe der Silikontenside,
- wobei die Gesamtmenge an erstem, zweitem und drittem Tensid im Bereich von 2 bis 6 Gew.-% liegt,
mit der Maßgabe, dass
- das Massenverhältnis des ersten Tensids zum zweiten Tensid 1,4 oder kleiner ist, wenn der Gehalt des dritten Tensids im Bereich von 0 bis weniger als 0,5 Gew.-% liegt und die Gesamtmenge an erstem und zweitem Tensid 2 Gew.-% oder weniger beträgt,
wobei sämtliche Gewichtsprozentangaben bezogen sind auf die Gesamtmasse der Zusammensetzung.

## Claims

1. Curable, one- or multicomponent composition for dental casting, comprising
- a curable base composition
and
- a surfactant system comprising or consisting of
- 0.75 to 3 wt.% of a first surfactant of the formula 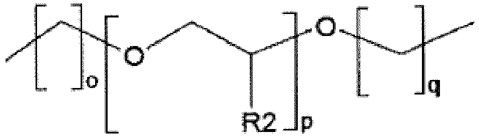 wherein
o is an integer from 5 to 22,
p is an integer from 2 to 20, and
q is an integer from 0 to 5
R2 is hydrogen or a methyl group, wherein within a molecule R2 can have different meanings in the context of the definition given,
- 0.75 to 3 wt.% of a second surfactant which is selected from the group of nonionic fluorosurfactants,
- 0 to less than 0.5 wt.% of a third surfactant which is selected from the group of silicone surfactants,
- wherein the total amount of first, second and third surfactant is in the range of from 2 to 6 wt.%,
with the proviso that
- the weight ratio of the first surfactant to the second surfactant is 1.4 or less when the content of the third surfactant is in the range of from 0 to less than 0.5 wt.% and the total amount of first and second surfactant is 2 wt.% or less, wherein all the per cent by weight data are based on the total weight of the composition.

2. Curable composition according to claim 1, wherein the composition is a multicomponent, preferably two-component system comprising a first component and a second component, the constituents of which are coordinated to one another such that curing is initiated by mixing the first and second components and the further components optionally present.

3. Curable composition according to one of the preceding claims, wherein the curable base composition is selected from the group of
- the organopolysiloxanes which crosslink by an addition reaction,
- the organopolysiloxanes which crosslink by a condensation reaction,
- the polyethers comprising aziridino radicals which crosslink by an addition reaction,
- the polyethers comprising alkenyl radicals which crosslink by an addition reaction and
- the polyethers comprising alkoxysilyl radicals which crosslink by a condensation reaction.

4. Curable composition according to claim 3, wherein the curable base composition is an organopolysiloxane which crosslinks by an addition reaction, comprising
- an organopolysiloxane having at least two ethylenically unsaturated groups
and
- an organohydrogenpolysiloxane.

5. Curable composition according to claim 4, wherein the composition is a multicomponent, preferably two-component system comprising
- a first component comprising an organopolysiloxane having at least two ethylenically unsaturated groups and a hydrosilylation catalyst
and
- a second component comprising the organohydrogenpolysiloxane and optionally an organopolysiloxane having at least two ethylenically unsaturated groups,
wherein the first surfactant and the second surfactant (and optionally further surfactants) are preferably contained in the first and/or the second component.

6. Curable composition according to one of the preceding claims, further comprising one or more fillers and optionally further additives.

7. Curable composition according to one of the preceding claims, wherein the second surfactant is a compound of the formula
R_{f}-(CₙH₂ₙ)-Y-(CₘH₂ₘ-O)_{y}-R¹,
wherein
- R_{f} is a radical of the formula CₓF₂ₓ₊₁,
- x is an integer from 1 to 30, preferably 2 to 18,
- n is an integer from 0 to 30, preferably 1 to 3,
- Y is -O- or -CO-O-, preferably -O-,
- m is an integer from 2 to 6, preferably 2 or 3, particularly preferably 2, wherein m within a molecule can assume different values in the context of the definition given,
- y is an integer from 1 to 60, preferably 1 to 25 and
- R¹ is hydrogen or a monovalent organic radical, preferably hydrogen, C₁-C₆-alkyl or phenyl, particularly preferably hydrogen or an alkyl radical.

8. Use of a composition according to one of the preceding claims as a dental casting system.

9. Method of producing a composition according to one of claims 1 to 7, comprising the steps:
- providing a curable base composition,
- providing a surfactant system comprising or consisting of
- 0.75 to 3 wt.% of a first surfactant of the formula 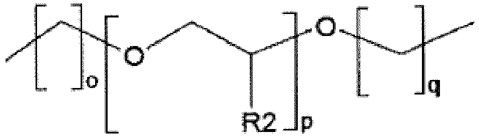 wherein
o is an integer from 5 to 22,
p is an integer from 2 to 20, and
q is an integer from 0 to 5
R2 is hydrogen or a methyl group, wherein within a molecule R2 can have different meanings in the context of the definition given,
- 0.75 to 3 wt.% of a second surfactant which is selected from the group of nonionic fluorosurfactants,
- 0 to less than 0.5 wt.% of a third surfactant which is selected from the group of silicone surfactants,
- wherein the total amount of first, second and third surfactant is in the range of from 2 to 6 wt.%,
with the proviso that
- the weight ratio of the first surfactant to the second surfactant is 1.4 or less when the content of the third surfactant is in the range of from 0 to less than 0.5 wt.% and the total amount of first and second surfactant is 2 wt.% or less,
wherein all the per cent by weight data are based on the total weight of the composition.

## Revendications

1. Composition durcissable à un ou à plusieurs composants servant à la prise d'empreinte dentaire, comprenant
- une composition de base durcissable
et
- un système tensioactif comprenant ou constitué
-- de 0,75 à 3 % en poids d'un premier agent tensioactif de la formule 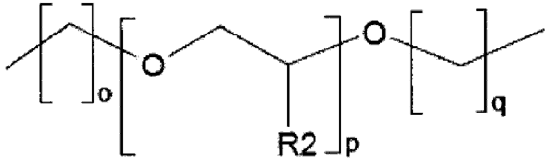 dans laquelle
o est un nombre entier allant de 5 à 22,
p est un nombre entier allant de 2 à 20, et
q est un nombre entier allant de 0 à 5 ;
R2 est de l'hydrogène ou un groupe méthyle, dans laquelle au sein de la molécule, R2 peut avoir différentes significations dans le cadre de la définition donnée,
-- 0,75 à 3 % en poids d'un deuxième agent tensioactif, qui est choisi parmi le groupe des agents tensioactifs fluorés non ioniques,
-- 0 à moins de 0,5 % en poids d'un troisième agent tensioactif, qui est choisi parmi le groupe des agents tensioactifs siliconés,
-- dans laquelle la quantité totale du premier, deuxième et troisième agent tensioactif se situe dans la plage allant de 2 à 6 % en poids
à la condition que
-- le rapport de masse entre le premier agent tensioactif et le deuxième agent tensioactif soit égal ou inférieur à 1,4 quand la teneur du troisième agent tensioactif se situe dans la plage allant de 0 à moins de 0,5 % en poids et que la quantité totale du premier et du deuxième agent tensioactif représente 2 % en poids ou moins, dans laquelle toutes les indications exprimées en pourcentage en poids se rapportent à la masse totale de la composition.

2. Composition durcissable selon la revendication 1, dans laquelle la composition est un système à plusieurs composants, de préférence à deux composants, comprenant un premier composant et un deuxième composant, dont les constituants sont assortis les uns aux autres de telle sorte que le durcissement est initié par le mélange du premier et du deuxième ainsi qu'éventuellement d'autres composants présents.

3. Composition durcissable selon l'une quelconque des revendications précédentes, dans laquelle la composition de base durcissable est choisie parmi le groupe
- des organopolysiloxanes à réticulation par réaction d'addition,
- des organopolysilocanes à réticulation par réaction par condensation,
- des polyéthers contenant des radicaux aziridino à réticulation par réaction d'addition,
- des polyéthers contenant des radicaux alcényle à réticulation par réaction d'addition.

4. Composition durcissable selon la revendication 3, dans laquelle la composition de base durcissable est un organopolysiloxane à réticulation par réaction par addition, comprenant
- un organopolysiloxane pourvu au moins de deux groupes éthyléniquement insaturés
et
- un organohydrogénopolysiloxane.

5. Composition durcissable selon la revendication 4, dans laquelle la composition est un système à plusieurs composants, de préférence un système à deux composants, comprenant
- un premier composant comprenant un organopolysiloxane pourvu au moins de deux groupes éthyléniquement insaturés ainsi qu'un catalyseur d'hydrosilylation
et
- un deuxième composant comprenant l'organohydrogénopolysiloxane et en option un organopolysiloxane pourvu au moins de deux groupes éthyléniquement insaturés,
dans laquelle le premier agent tensioactif et le deuxième agent tensioactif (ainsi qu'éventuellement d'autres agents tensioactifs) sont contenus de préférence dans le premier et/ou dans le deuxième composant.

6. Composition durcissable selon l'une quelconque des revendications précédentes, comprenant par ailleurs une ou plusieurs charges ainsi qu'éventuellement d'autres additifs.

7. Composition durcissable selon l'une quelconque des revendications précédentes, dans laquelle le deuxième agent tensioactif est un composé de la formule
R_{f}-(CₙH₂ₙ)-Y-(CₘH₂ₘ-O)_{y}-R¹,
dans laquelle
- R_{f} est un radical de la formule CₓF₂ₓ₊₁,
- x est un nombre entier allant de 1 à 30, de préférence de 2 à 18,
- n est un nombre entier allant de 0 à 30, de préférence de 1 à 3,
- Y est -O- ou -CO-O, de préférence -O-,
- m est un nombre entier allant de 2 à 6, de préférence égal à 2 ou 3, de manière préférée égal à 2, dans laquelle m peut prendre, au sein d'une molécule, dans le cadre de la définition donnée, différentes valeurs,
- y est un nombre entier allant de 1 à 60, de préférence de 1 à 25, et
- R¹ est de l'hydrogène ou un radical organique monovalent, de préférence de l'hydrogène, de l'alkyle en C₁-C₆ ou du phényle, de manière particulièrement préférée de l'hydrogène ou un radical alkyle.

8. Utilisation d'une composition selon l'une quelconque des revendications précédentes, en tant que système de prise d'empreinte dentaire.

9. Procédé servant à fabriquer une composition selon l'une quelconque des revendications 1 à 7, comprenant les étapes suivantes consistant à :
- fournir une composition de base durcissable
et
- fournir un système tensioactif comprenant ou constitué
-- de 0,75 à 3 % en poids d'un premier agent tensioactif de la formule 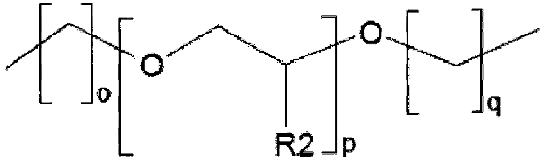 dans laquelle
o est un nombre entier allant de 5 à 22,
p est un nombre entier allant de 2 à 20, et
q est un nombre entier allant de 0 à 5 ;
R2 est de l'hydrogène ou un groupe méthyle, dans lequel au sein de la molécule, R2 peut avoir différentes significations dans le cadre de la définition donnée,
-- 0,75 à 3 % en poids d'un deuxième agent tensioactif, qui est choisi parmi le groupe des agents tensioactifs fluorés non ioniques,
-- 0 à moins de 0,5 % en poids d'un troisième agent tensioactif, qui est choisi parmi le groupe des agents tensioactifs siliconés,
-- dans laquelle la quantité totale d'un premier, un deuxième et un troisième agent tensioactif se situe dans la plage allant de 2 à 6 % en poids
à la condition que
-- le rapport de masse entre le premier agent tensioactif et le deuxième agent tensioactif soit égal ou inférieur à 1,4 quand la teneur du troisième agent tensioactif se situe dans la plage allant de 0 à moins de 0,5 % en poids et que la quantité totale du premier et du deuxième agent tensioactif représente 2 % en poids ou moins,
dans lequel toutes les indications exprimées en pourcentage en poids se rapportent à la masse totale de la composition.
